# EUROPEAN PATENT APPLICATION

(11) **EP 1 468 702 A1**
(43) Date of publication of application: **20.10.2004**
(21) Application number: 03425223.9
(22) Date of filing: 09.04.2003
(51) Int. Cl.: A61L 9/03, A61L 9/12

(54) **Device for diffusing substances in an environment**

(71) Applicant: Creative Company S.r.l., 24040 Pognano (Bergamo) (IT)
(72) Inventor: Necchi, Andrea Giuliano, 24045 Fara Gera d'Adda (Bergamo) (IT)
(74) Representative: Siniscalco, Fabio

(57) **Abstract**

A device (1) for diffusing substances in an environment, comprising an electronic control circuit (8a) for controlling at least one operating parameter of the device. The device includes also electronic means (RW_IC) for detecting information corresponding to distinct operating conditions of the device and stored in respective memorization means (TR) that can be interchangeably associated with the device (1). The control circuit is connected to the detection means for controlling at least one operating parameter on the basis of the information detected in one of said memorization means.

## Description

The present invention concerns the diffusion of aromatic, perfumed and similar substances in environments and, more particularly, describes a device for diffusing substances in an environment.

Devices for the diffusion of fragrances or substances in environments are known and have been used for a long time both in the home and personal domain and in public environments, including - for example - offices, gymnasia, schools and hospitals.

These devices are employed, for example, to cover unpleasant odours and/or to diffuse anti-bacterial or disinfectant substances. Recently these devices have also been used, in accordance with a discipline known as aromatherapy, for diffusing aromatic substances in environments for the purpose of influencing or modifying the human mental state, for example, to facilitate concentration, learning, relaxation and also to increase sales.

The diffusion devices of conventional type heat a substance - a perfumed substance, for example - to stimulate the emission of its vapours into the environment. Devices of this type, especially when intended for use in large-size environments, usually also avail themselves of a ventilation system to assure better and more rapid diffusion of the vapours emitted by the substance.

The most recent diffusion devices are provided with control electronics to program a time for bringing the device into operation and a time for shutting it down and are also equipped with a manual regulation device that enables a user to vary the speed of rotation of a fan of the ventilation system.

With reference to the devices known to prior art it should be noted that, notwithstanding their widespread use, they are associated with the drawback of having a limited and primitive possibility of regulation the operational parameters and for this reason are not always capable of assuring optimised diffusion of the substance into the environment. Furthermore, the devices known. to prior art do not comprise any precaution for avoiding the diffusion of non-controlled substances.

The problem underlying the present invention is that of making available a diffuser for environments that has structural and functional characteristics such as to avoid the aforementioned drawbacks associated with prior art.

This problem is solved by means of a diffuser device in accordance with Claims 1 to 10 attached hereto.

The object of the present invention also comprises a container as described in Claims 11 to 20 hereinbelow and the use of this container in the diffuser device.

Further characteristics and advantages of the invention will be understood more readily from the detailed description about to be given of its preferred embodiments, which is given purely by way of example, so that it is not to be considered limitative in any way, and makes reference to the attached drawings, of which:
- Figure 1 shows a schematic perspective view of a diffuser in accordance with the inventions,
- Figure 2 shows a perspective view of the diffuser of Figure 1 with its outer cover detached,
- Figure 3 shows a schematic functional diagramme of a control circuit that can be used in the diffuser of Figure 1.

Referring to the figures attached hereto, there will now be described a preferred and exemplifying embodiment of a diffuser in accordance with the invention.

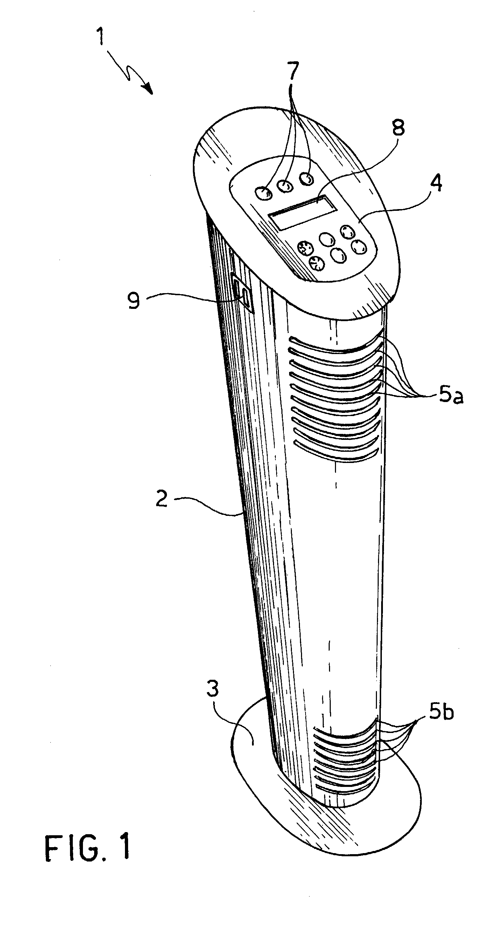

Figure 1 shows a perspective view of a diffuser 1 for diffusing substances in an environment, for the sake of simplicity hereinafter referred to also by the term diffuser device or diffuser.

Without thereby introducing any limitation, the diffuser 1 described hereinafter is suitable for being used for diffusing the vapours of a substance in environments of medium and large size (for example, having volumes > 100 m³).

In the present example and not as a limitation, the substance to be diffused is a liquid substance, for example, an essential oil diluted in water. Other choices are however possible and the substance to be diffused could be, alternatively, a gel, a powder, a granular material or a gas.

In the example the substance to be diffused is a perfumed substance, alternatively or in addition thereto the substance is an antibacterial and/or disinfecting substance and/or a substance having a repellent effect for insects or other animals.

The diffuser 1 comprises a casing, for example in the form of a tubular column 2, preferably having a substantially cylindrical or elliptical section, resting on a base 3. The tubular column casing 2, or more simply the casing 2, comprises preferably one or more upper openings 5a and one or more lower openings 5b.

The lower openings 5b, for example, are such as to constitute an inlet section that permits the air to enter the diffuser 1, while the upper openings 5a, for example, are such as to constitute an outlet section for the air containing the diffused substance.

In a particularly advantageous embodiment the upper openings 5a and the lower openings 5b are set an appropriate distance apart, for example arranged substantially in proximity of the opposite ends of the casing 2, in such a way as to define a duct for the circulation of the air within the casing 2.

The diffuser 1 also comprises a control panel 4, for example arranged substantially at the top of the casing 2, preferably with input buttons 7 and a display device 8 that is, for example, a liquid crystal pixel-matrix screen.

In a preferred embodiment the diffuser 1 comprises at least one preferably bi-directional communication and/or programming interface 9.

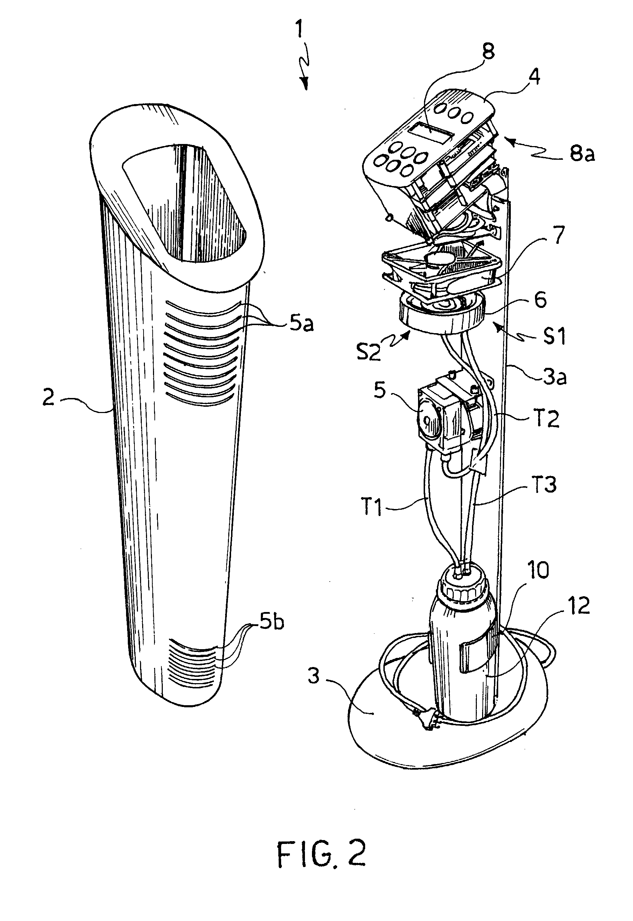

Figure 2 shows a view of the diffuser 1 with the casing 2 detached from the rest of the diffuser.

In the example of Figure 2 the diffuser 1 comprises an upright 3a, preferably in the form of metallic stirrup, fixed to the supporting base 3 and substantially perpendicular thereto.

Furthermore, the diffuser 1 comprises, preferably fixed to the upright 3a, several items of equipment functional for the diffusion. In the example of Figure 2 this equipment comprises, stacked from the base upwards, a dosing device 5, an evaporation recipient 6 and at least one electric fan 7.

The diffuser 1 further comprises an electronic control circuit 8a.

Advantageously, the equipment functional for diffusion purposes (in the example, the dosing device 5, the evaporation recipient 6, the fan 7) and the control panel 4 are electrically connected to the electronic control circuit 8a.

The dosing device 5 makes possible the transfer of the substance to be diffused, contained within a container 12, from the substance container 12 to the evaporation recipient 6.

In Figure 2 the substance container 12 is a bottle 12 comprising an outer shell to contain the substance. The bottle 12 can be removably associated with the diffuser 1 by connection means integral with the upright 3a.

Preferably, the connection means comprise a C-shaped portion having two curvilinear arms 10 capable of at least partially surrounding the container 12. The container 12 may be, for example, an interchangeable bottle.

The dosing device 5 preferably includes a peristaltic pump that can be connected to the bottle 12 containing the substance to be diffused and to the evaporation recipient in a conventional manner by means of the flexible hoses T1 and T2.

In a preferred embodiment the evaporation recipient 6 can be electrically heated and comprises an inlet section S1 to receive the substance from the dosing device 6 through the hose T2.

More preferably, the evaporation recipient 6 also comprises an outlet section S2 to permit the part of the substance that has not been evaporated to return to the bottle 12 through a flexible return hose T3.

Preferably, the evaporation recipient 6 has associated with it a passive heater that exploits the heat of the evaporation recipient for heating a second substance (a propolis, for example).

It should be noted that the particular arrangement of the inlet openings 5a and the outlet openings 5b renders possible a better exploitation of the space within the casing 2, i.e. the circulation duct for the air, by inserting between inlet section and the outlet section of the air such other optional items of equipment and accessories functional for diffusing the substance as, for example, a diaphragm, a UV antibacterial lamp, a filter, and the like.

It should be noted that this accessory equipment could also comprise optional expansion modules that, for example, could be installed some time after the sale of the diffuser.

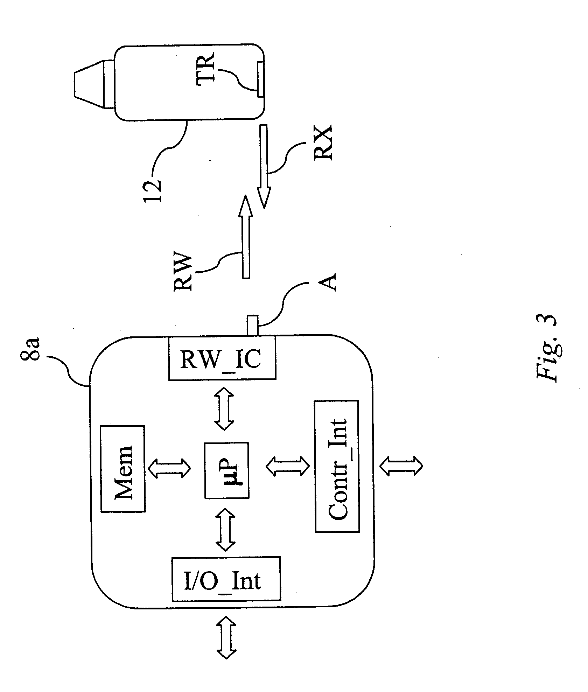

Figure 3 illustrates a layout example of the control circuit 8a and the principle of interaction with the substance to be diffused contained in the bottle 12.

It should be noted that, according to the example of Figure 3, the bottle 12 is mechanically coupled with memorization means TR that store information capable of establishing a particular operating condition of the diffuser 1. In other words, said memorization means TR store data useful for controlling the diffuser 1 by means of the control circuit 8a.

For example, said data serve to identify the substance or comprise such other operating parameters of the equipment functional for diffusion purposes as, for example, the temperature of the evaporation recipient and/or the speed of rotation of the fan or, more generally, a combination, which could even be complex, of operating parameters that optimise the functioning of the diffuser 1 in accordance with the substance in the container 12.

In a particularly advantageous embodiment, moreover, the information stored in the memorization means TR is encoded or encrypted.

Preferably, the memorization means TR comprise a conventional transponder, for example, a passive transponder (i.e. capable of being operated by radio-frequency energy that strikes it).

A transponder of this type is an integrated circuit that includes a non-volatile memory (for example, ROM, Flash or EEPROM), a receiver-transmitter set provided with an antenna and an appropriate control or processing circuit (not shown).

Preferably, in the case in which the container 12 is in the form of a bottle, the transponder TR is a flat discoidal body of small size (for example, having a diameter of 20 mm and a thickness of 2-3 mm) mechanically coupled (i.e. fixed, glued, riveted, etc) to the bottom of the bottle 12, either inside or outside the bottle shell. A transponder of this type is produced, for example, by Messrs. SOKYMAT SA and marketed under the name of "World Tag".

As an alternative to the transponder, it is also possible to use memorization means of a different kind, for example, a bar code or a magnetic band.

As shown in Figure 3, the electronic control circuit 8a, which could be, for example, a digital electronic board, will advantageously comprise at least one processing unit µP (a microprocessor, for example), at least one memory unit Mem, an input/output interface section I/O_Int and a control interface Contr_Int.

In a preferred embodiment the memory unit Mem permits both the memorization of data and the memorization of control software.

Preferably, the interface section I/O_Int connects the electronic control circuit 8a to the communication /programming interface 9 of the diffuser 1 (which may be, for example, an RS-232 gate or an RS-485 gate) to permit downloading and updating of the control software and/or connecting the diffuser 1 to a local network, etc.

The control interface Contr_Int, on the other hand, makes it possible for the control circuit 8a to control the functioning of the diffuser 1, that is to say, establish its operating conditions by controlling such operating parameters of the equipment function for diffusion as, for example, the switching on and off and the speed of rotation of the fan 7, the switching on and off and the temperature of the evaporation recipient 6, the switching on and off and the speed of the peristaltic pump 5, etc.

The diffuser 1 also includes electronic detection means RW_IC capable of automatically detecting and reading the information stored in the memorization means TR, in the present example associated with the particular bottle 12 of the substance to be diffused.

In the particular case in which the memorization means TR comprise a transponder, the detection means RW_IC are realized by means of a common integrated device known by the name of "transponder reader". A device of this type is substantially a receiver-transmitter of radio-frequency signals comprising preferably an antenna A and an integrated circuit RW_IC, for example, in CMOS technology. This receiver-transmitter makes it possible to send radio-frequency interrogation signals RW to the transponder TR and to receive radio-frequency response signal RX from the transponder.

When different memorization means are used (bar code, magnetic band), the choice of appropriate detection means is obvious to a person skilled in the art.

The electronic detection means RW_IC are connected to the electronic control circuit 8a and the latter is such as to control at least one operating parameter connected with the functioning of the diffuser 1 on the basis of the information detected by the detection means RW_IC.

Preferably, the electronic detection means RW_IC are comprised in the electronic control circuit 8a.

As already mentioned, in a preferred embodiment the information stored in the memorization means TR comprises at least one data item that identifies the substance to be diffused, for example a digital code that univocally identifies the substance, and the stored information does not comprise the control data.

In this case the control data are memorized locally in the memory unit Mem of the electronic control circuit 8a.

For example, the data item that identifies the substance to be diffused is a digital code that univocally identifies the substance and the memory unit Mem contains a list of identification data (digital codes in the example) and for each of these includes a data structure that memorizes control data. For example, for each identification code the memory unit Mem contains a respective data structure that locally memorizes a plurality of such operating parameters as the temperature of the evaporation recipient and/or the speed of rotation of the fan or, more generally, a combination of operating parameters, even a complex one, that optimize the functioning of the diffuser 1 for diffusing the substance associated with the detected identification data item.

In an alternative embodiment, the information stored in the memorization means TR comprises the control data for diffusing the substance.

For example, given the latter variant, when the substance is inserted in the diffuser 1, the control circuit 8a is such as to automatically detect, i.e. to read control data from memorization means TR associated with the substance, for example, such operating parameters as the temperature of the evaporation recipient and/or the speed of rotation of the fan or, more generally, a combination, even a complex one, of operating parameters that optimise the functioning of the diffuser 1 on the basis of the substance to be diffused.

In a particularly advantageous embodiment the detection means RW_IC also comprise writing means to modify at least a part of the information contained in the memorization means TR.

In greater detail, given the particular case in which the detection means read information memorized in a transponder TR, these means are also provided with radio-frequency writing means of the transponder TR to modify the information contained in it.

In this case the detection means are realized by means of a conventional radio-frequency reading/writing circuit for transponders.

In a particular application, for example, the detection means are such as to read from the transponder TR, by means of interrogation signals RW, a data item correlated with the maximum utilization time of a container 12 of the substance.

Advantageously, the transponder TR stores a data item regarding the residual utilization time of the substance container and the writing means of the transponder make it possible to update (in real time, for example) this data item in the transponder TR.

Referring to the attached drawings, there will now be given a brief explanation of the functioning of the diffuser 1 in accordance with the present invention in the particular case described above in which the memorization means comprise a transponder TR.

In the phase of producing or confectioning a particular substance to be diffused, the producer writes into the transponder TR information regarding the diffusion of a substance and couples, i.e. associates, the transponder TR with the container 12 of the substance.

For example, the producer writes into the transponder TR control data, for example, such operating parameters that make it possible to obtain an optimal diffusion of the substance as:
- data regarding the timing of the dosing device 5, including - for example - the time interval between two successive dosings and the duration of each dosing;
- data regarding the temperature of the evaporation recipient 6;
- data regarding the speed of the fan 7 and the timing of the fan;
- data regarding the maximum utilization time of the bottle 12, for example, measured in hours and minutes.

According to his own particular needs, a user of the diffuser 1 acquires a container 12 filled with the substance, a bottle 12 for example, for use in a diffuser 1 and inserts this bottle in the diffuser in a simple and rapid manner.

Following the insertion of the bottle 12 in the diffuser 1, the detection means RW_IC interrogate the transponder TR associated with the bottle 12 of the substance by sending an interrogation signal RW, for example, every time the diffuser is started up.

The transponder TR responds to the interrogation signal RW by sending to the detection means a response signal RX containing the control data for the diffusion of the substance contained in the bottle 12. From this the electronic control circuit 8a connected to the detection means RW_IC receives the control data contained in the response signal and stores them in the memory unit Mem.

In an automatic functioning mode the diffuser 1 operates in full autonomy by making use of the data stored in the memory unit Mem.

In greater detail, the electronic control circuit 8a regulates the functioning of the dosing device 5, the evaporation recipient 6 and the fan 7 in accordance with these data.

In another functioning mode, however, the user, availing himself of the control panel 4 and display device 8, can decide to modify the operating parameters of the diffuser 1 or any part thereof either temporarily or permanently.

In a very advantageous embodiment, the detection means RW_IC read the data item regarding the maximum utilization time from the transponder TR during the functioning of the diffuser 1 and update the data item in question on the basis of the effective utilization time of the substance.

In this way the transponder TR contains a data item regarding the residual utilization time of the substance, which is then gradually reduced as the substance is diffused. The data item in question may be, for example, a value of the residual time expressed in hours and minutes.

When this value is close to zero, the user is informed by means of, for example, a "Close to exhaustion" notice on the display device 8.

When the value actually reaches zero, the user is informed by means of, for example, an "Error: substance exhausted" notice and the electronic control circuit 8a ignores any attempt to set the diffuser in motion, thus preventing the diffuser 1 from functioning.

As will readily be appreciated from the above description, the diffuser in accordance with the invention makes it possible to satisfy the aforesaid needs and to obviate the difficulties mentioned in connection with the prior art.

As explained, in fact, the diffuser in accordance with the invention assures an optimal diffusion of the substance without involving the user in complicated operations of calibration, programming and regulation.

It should be noted that a diffuser in accordance with the invention is suitable for being used with a vast range of substances of which the diffusion can call for a wide range of different operating parameters.

For example, there are substances that, having a very intense perfume, call for a temperature of the evaporation recipient and dosing well below the corresponding parameters associated with weaker and more delicate fragrances.

It should therefore be noted that the operating conditions of the diffuser 1 vary on the basis of the control data contained in the memorization means, the transponder for example. Since these memorization means may be interchanged by the user by, for example, changing the substance to be diffused and/or its container, the operating conditions of the diffuser may be varied without a direct intervention of the user intended to regulate the diffuser parameters.

It should also be noted that whenever the diffuser users inform the producer of a preference for operating parameters that differ from the functioning modality preset by the producer, the latter may vary the control data in a very simple manner and with the greatest transparency for the user by, for example, modifying the operating parameters memorized in the transponder TR.

On the other hand, in the case in which the transponder TR contains the identification code of the substance rather than the operating parameters, the same result can be obtained by remote updating - via the communication / programming interface 9 - of the local control data present in the memory unit Mem of the diffuser 1.

Another thing to be noted is that the writing means that update the data memorized in the transponder TR by updating the data item regarding the maximum or residual utilization time of the substance have the important advantage of automatically informing the user of the need for inserting a new bottle of substance in the diffuser.

Furthermore, advantageously for the producer of the diffuser or the substance, thanks to this particular aspect, the user could not acquire substance reloads produced by third parties, substance counterfeiters for example, and, above all, any attempt to refill an empty bottle with new substance would not be attended by success, because the data item causing the system to signal zero residual time would remain preserved in the transponder.

It should further be noticed that counterfeited reloads could prove to be dangerous for health whenever the place of tested components - for example, components having a substantially natural base - is taken by cheaper but toxic synthetic substances.

Advantageously, a diffuser in accordance with the present invention, thanks to the presence of memorization means associated with the substance, also makes it possible to avoid the diffusion of such substances not intended for diffusion as, for example, substances used for washing the diffuser.

It should be noted that the particular embodiment that envisages the use of a transponder and reading /writing means of said transponder, offers the aforesaid advantages without significantly affecting the cost of the diffusers and the reloads of the substances to be diffused.

Lastly, it should be noted that, even though the described diffusers is of the type known by the name of column diffuser, the teachings of the present invention can be applied also to diffusion devices that are different in nature or shape. For example, a diffuser in accordance with the present invention could be realized, with just a few adaptations, as a module to be inserted in the final stage of a (domestic or industrial) air conditioning system for the purpose of diffusing the vapours of a perfumed, antibacterial or other substance as the output of the system.

Obviously, with a view to satisfying contingent or specific needs, a person skilled in the art could introduce numerous modifications and variants into the diffusion device described hereinabove without for that reason overstepping the scope of the invention as defined by the claims set out below.

## Claims

1. A device (1) for diffusing substances in an environment, comprising an electronic control circuit (8a) for controlling at least one operating parameter of the device, **characterized in that** it includes also electronic means (RW_IC) for detecting information corresponding to distinct operating conditions of the device and stored in respective memorization means (TR) that can be interchangeably associated with the device (1), the control circuit being connected to the detection means for controlling said at least one operating parameter on the basis of the information detected in one of said memorization means.

2. A device (1) in accordance with Claim 1, wherein the information stored in the memorization means (TR) comprises at least one data item identifying a substance to be diffused and wherein the control circuit includes a memory unit (Mem) containing local control data for the diffusion of the substance associated with said identification data item, the electronic control circuit (8a) being such as to control the at least one operating parameter on the basis of these local control data.

3. A device (1) in accordance with Claim 1, wherein the information stored in the memorization means (TR) comprise control data for the diffusion of a substance, the electronic control circuit (8a) being such as to control the at least one operating parameter on the basis of these control data.

4. A device (1) in accordance with Claim 1, wherein the detection means (TR) comprise writing means to modify at least part of the information stored in the memorization means (TR).

5. A device (1) in accordance with Claim 1, wherein the detection means (RW_IC) comprise a radio-frequency reading device and the memorization means (TR) comprise a transponder (TR).

6. A device (1) in accordance with Claim 5, wherein said transponder (TR) is coupled with a substance container (12).

7. A device (1) in accordance with Claim 6, wherein the substance is a perfumed and/or antibacterial and/or disinfectant substance and/or a substance that repels insects or other animals.

8. A device (1) in accordance with any one of the preceding claims, comprising also a plurality of equipment functional for the diffusion and wherein said plurality includes at least one dosing device (5), at least one evaporation recipient (6) and at least one fan (7) electrically connected to the electric control circuit (8a) and wherein said dosing device makes it possible to transfer of substance to be diffused, contained in a container (12), from the container (12) to the evaporation recipient (6).

9. A device in accordance with Claim 8, comprising a casing (2) that accommodates (houses) said equipment, said casing including at least one first (5a) and at least one second (5b) opening for, respectively, the entry of the air and the exit of the air containing the substance.

10. A device (1) in accordance with Claim 9, wherein said first and second opening are separated from each other in such a way as to define a circulation duct for the air within said casing (2), of such a length as to permit the installation within said duct of optional equipment functional for diffusion.

11. A container of a substance to be diffused in an environment by means of a diffuser device, the container comprising an outer shell (12) for containing said substance, **characterized in that** the shell is mechanically coupled to memorization means (TR) such as to store information relating to an operating condition of the diffuser device, the memorization means being capable of being associated with a diffuser device (1) realized in accordance with at least one of the preceding claims.

12. A container in accordance with Claim 11, wherein said information stored in the memorization means comprises at least one data item identifying the substance to be diffused.

13. A container in accordance with Claim 11, wherein said information stored in the memorization means includes a data item relating to the maximum time the container can be utilized by the diffuser device.

14. A container in accordance with Claim 11, wherein the memorization means make it possible to modify and update by means of the writing means included in the diffuser device (1) at least part of the information stored in them.

15. A container in accordance with Claims 13 and 14, wherein a data item relating to a residual utilization time of the substance contained in the shell is stored in the memorization means, said data item relating to the residual utilization time being updatable by means of said writing means.

16. A container in accordance with Claim 11, wherein said memorization means are fixed to the shell of the container (12).

17. A container in accordance with Claim 11, wherein said memorization means are outside the shell of the container (12).

18. A container in accordance with Claim 11, wherein said memorization means comprise a transponder that includes an integrated circuit provided with a non-volatile memory for storing the information corresponding to an operating condition.

19. A container in accordance with Claim 18, wherein said transponder includes a receiver-transmitter set provided with an antenna to receive interrogation signals from said detection means and to transmit to the detection means response signals (RX) relating to said information and an appropriate control / processing circuit.

20. A container in accordance with Claim 19, wherein said transponder is of the passive type, i.e. can be supplied by radio-frequency energy emitted by said detection means.

21. Use of a container in accordance with at least one of Claims 11 to 20 for a diffuser device realized in accordance with at least one of Claims 1 to 10.
